# EUROPEAN PATENT SPECIFICATION

(11) **EP 1 682 927 B1**
(45) Date of publication and mention of the grant of the patent: **27.12.2017**
(21) Application number: 04813481.1
(22) Date of filing: 09.11.2004
(51) Int. Cl.: A61B 3/00, A61B 3/10, A61F 9/008, A61B 18/20

(54) **METHODS AND DEVICES FOR TESTING TORSIONAL ALIGNMENT BETWEEN A DIAGNOSTIC DEVICE AND A LASER REFRACTIVE SYSTEM**
VERFAHREN UND EINRICHTUNGEN ZUR PRÜFUNG DER TORSIONSAUSRICHTUNG ZWISCHEN EINER DIAGNOSEEINRICHTUNG UND EINEM LASERBRECHUNGSSYSTEM
PROCEDES ET DISPOSITIFS DE TEST DE L'ALIGNEMENT EN TORSION D'UN DISPOSITIF DE DIAGNOSTIC AVEC UN SYSTEME A REFRACTION LASER

(30) Priority: 10.11.2003 US 518826 P
(43) Date of publication of application: 26.07.2006
(73) Proprietor: AMO Manufacturing USA, LLC, Santa Ana, CA 92705-4933 (US)
(72) Inventor: CAMPBELL, Charles E., Berkeley, California 94705 (US); CHERNYAK, Dimitri, Santa Clara, California 95051 (US)
(74) Representative: Clark, Jane Anne
(86) International application number: PCT/US2004/041166
(87) International publication number: WO 2005/047938

(56) References cited:
- WO-A1-01/28476
- WO-A2-01/89373
- WO-A2-02/064031
- WO-A2-2004/089214
- US-A- 5 713 893
- US-A- 5 713 893
- US-A1- 2002 026 181
- US-A1- 2003 120 266
- US-A1- 2003 120 266
- US-A1- 2003 223 037

## Description

### CROSS-REFERENCES TO RELATED APPLICATIONS

The present application claims the benefit of U.S. Patent Application No. 60/518,826, filed on November 10, 2003.

### BACKGROUND OF THE INVENTION

The present application relates generally to methods and devices for testing the accuracy of a torsional misalignment measurement between two torsionally misaligned images. More specifically, the present invention relates to methods and devices that are used to compare a measured torsional misalignment to an actual torsional misalignment between two different images taken of the test device.

Known laser eye procedures generally employ an ultraviolet or infrared laser to remove a microscopic layer of stromal tissue from the cornea of the eye to alter the refractive characteristics of the eye. The laser removes a selected shape of the corneal tissue, often to correct refractive errors of the eye. Ultraviolet laser ablation results in photo-decomposition of the corneal tissue, but generally does not cause thermal damage to adjacent and underlying tissues of the eye. The irradiated molecules are broken into smaller volatile fragments photochemically, directly breaking the intermolecular bonds.

Laser ablation procedures can remove the targeted stroma of the cornea to change the cornea's contour for varying purposes, such as for correcting myopia, hyperopia, astigmatism, and the like. Control over the distribution of ablation energy across the cornea may be provided by a variety of systems and methods, including the use of ablatable masks, fixed and moveable apertures, controlled scanning systems, eye movement tracking mechanisms, and the like. In known systems, the laser beam often comprises a series of discrete pulses of laser light energy, with the total shape and amount of tissue removed being determined by the shape, size, location, and/or number of a pattern of laser energy pulses impinging on the cornea. A variety of algorithms may be used to calculate the pattern of laser pulses used to reshape the cornea so as to correct a refractive error of the eye. Known systems make use of a variety of forms of lasers and/or laser energy to effect the correction, including infrared lasers, ultraviolet lasers, femtosecond lasers, wavelength multiplied solid-state lasers, and the like. Alternative vision correction techniques make use of radial incisions in the cornea, intraocular lenses, removable corneal support structures, thermal shaping, and the like.

Known corneal correction treatment methods have generally been successful in correcting standard vision errors, such as myopia, hyperopia, astigmatism, and the like. However, as with all successes, still further improvements would be desirable. Toward that end, diagnostic devices, such as wavefront measurement systems are now available to measure the refractive characteristics of a particular patient's eye. By customizing an ablation pattern based on wavefront measurements, it may be possible to correct minor refractive errors so as to reliably and repeatably provide visual accuities greater than 20/20. Alternatively, it maybe desirable to correct aberrations of the eye that improve visual acuity to better than 20/20. Unfortunately, these measurement systems are not immune from measurement error. Similarly, the calculation of the ablation profile, the transfer of information from the measurement system to the ablation system, and the operation of the ablation system all provide opportunities for the introduction of errors, so that the actual visual accuities provided by real-world wavefront-based correction systems may not be as good as might be theoretically possible.

For example, one potential problem with the use of wavefront measurements is aligning the customized laser ablation pattern that is based on the wavefront measurements with the real-time position of the patient's eye during the ablation treatment. In order to achieve precise registration between the customized ablation pattern delivered to the patient's eye, the real-time position of the eye should share a common coordinate system with the ablation pattern. However, in order to provide the common coordinate system, the software algorithms of the laser refractive system should be able to accurately locate and track the position and cyclotorsional orientation of the patient's eye.

In light of the above, it would be desirable to provide methods and test devices that maybe used to measure the accuracy of the tracking algorithms of the laser refractive system.

WO 02/064031 describes multidimensional eye tracking and position measurement system for diagnosis and treatment of the eye.

US 2003/0120266 describes an ophthalmic apparatus for obtaining differences between the pupil positions in photopia and scotopia accurately, under corneal surgery apparatus for ablating a cornea of an eye by laser beam irradiation allowing for those differences. US 5713893 describes a test substrate for the calibration of a laser used in ophthalmic surgery. The test substrate is formed form a homogenous relatively anhydrous material that ablates at a rate approximately that of human corneal tissue. So provides a substrate that can be analyzed pre-ablation and post-ablation so as to allow evaluation of the ablating laser.

### BRIEF SUMMARY OF THE INVENTION

The present invention is set out in the appended claims. Described herein are methods, systems, and test devices for measuring an accuracy of a cyclotorsional algorithm. In particular, as described the accuracy of the cyclotorsional algorithm is calculated by comparing a known torsional misalignment angle between a test device in two images to a calculated torsional misalignment angle between the test device two images that is calculated by the cyclotorsional algorithm.

In one aspect, there is described a test device for testing an accuracy of a misalignment measurement between a diagnostic device and a laser refractive system. The device comprises a body comprising a proximal portion and a distal portion. The proximal portion defines a radiused corneal surface and an iris, and the distal portion defines a retinal surface. The test devices typically have visual and optical characteristics that are similar to a human eye. Consequently, the test devices
may be used with existing commercial diagnostic devices and laser refractive systems without having to alter the diagnostic devices and laser refractive systems.

Embodiments of the test device are typically comprised of a material that has similar optical characteristics to a human eye. In one embodiment, the test device is composed of cast polymethylmethacrylate (PMMA). PMMA has dispersion characteristics that are similar to that of the human eye. As can be appreciated, the test devices of the present invention may be composed of other optical materials. For example, other material that may be used, include but are not limited to, glass, other plastics, or any optically clear material that can be ablated with a UV laser.

The iris of the test device is typically an annular region of the proximal portion that is substantially adjacent the radiused corneal surface. The iris surface may comprise one or more texture patches or markings that provides a unique marking that allows the cyclotorsional algorithm to identify the iris and thereafter determine the torsional offset between the two images of the test device. The iris texture patches may be created during manufacturing by scratching, scraping or otherwise processing the iris surface to create radial striations or other markings on the iris surface.

The retinal surface positioned in the distal portion of the test device typically defines a diffuse surface that absorbs light. In one embodiment, the retinal surface is manufactured to be optically "flat." The retinal surface may thereafter be polished to create an optically diffuse surface. Thereafter, a material may be applied to the diffuse surface to provide the light absorbing characteristics. One material that may be applied is a flat dark gray paint.

Optionally, the test device may comprise an alignment reference that allows the user to visually assess the torsional rotational offset of the test device in the images of the test device. The alignment reference may also be used by the cyclotorsional algorithms of the present invention to determine the torsional offset between the alignment reference in the images of the test device. The alignment reference may take on a variety of forms. In one embodiment, the alignment reference comprises an alignment pin that is positioned in a hole in the test device. The alignment pin may extend radially from a longitudinal axis of the test device and is viewable in the images of the test device. In other embodiments, the alignment reference may take the form of a flattened surface, a protrusion, marking, or the like.

In another aspect, there is described a method that comprises obtaining an image of the test device with the diagnostic device. To obtain the first image, the test device may be positioned within an optical axis of the diagnostic device in a first orientation. A second image of the test device may be obtained with the laser refractive system by positioning the test device in a second orientation within an optical axis of a laser refractive system. The second orientation may be in a torsionally offset orientation from the first orientation so as to provide a known torsional misalignment. The first and second images of the test device are compared to provide a measured torsional misalignment between the images of the test device. The measured torsional misalignment is compared to the known misalignment to determine the accuracy of the measured misalignment.

The described methods may (1) determine the cyclotorsional rotational offset between the diagnostic devices and the laser refractive system, (2) measure the ability of the algorithm to locate and match iris markers in the test device, and/or (3) demonstrate cyclotorsional compensation by removing high order aberrations from the test device.

In some embodiments, the diagnostic device is used to obtain measurement of the aberrations of the eye and may be an autorefractor or an aberrometer such as a wavefront sensor. For example, aberrometers using a Hartmann-Shack device, Tscherning device, ray-tracing technology and the like may be employed.

In another aspect, there is described a method of manufacturing a test device. The method comprises providing a body that comprises a first optical surface and a second, radiused optical surface. The body is treated to form a textured annular iris surface. The first optical surface is treated to create a pupil surface.

The textured annular iris surface may include unique patterns of striations and imperfections and may be created by polishing, scraping or otherwise enhancing the iris surface.

To create the pupil surface, the first optical surface may be polished to produce a diffuse back scattering surface and a material that absorbs light may be applied to the polished surface. For example, in one configuration, an 0.3 micron Al₂O₃ grit may be used to polish the first optical surface and a flat dark gray paint may be applied to the polished surface.

In a further aspect, there is described a kit. The kit may include any of the test devices described herein. The kits may further include instructions for use setting forth any of the methods described herein. The kits may also include packaging suitable for containing the test device and the instructions for use. Exemplary packaging includes pouches, trays, boxes, tubes, and the like. The instructions for use may be provided on a separate sheet of paper or other medium. Optionally, the instructions may be printed in whole or in part on the packaging.

These and other aspects of the invention will become more apparent from the following detailed description of the invention when taken in conjunction with the accompanying exemplary drawings.

### BRIEF DESCRIPTION OF THE DRAWINGS

FIGS. 1 and 1A illustrate different views of one embodiment of a test device.
FIG. 1B illustrates a geometry of one test device.
FIG. 2 schematically illustrates a simplified method.
FIG. 3 illustrates one embodiment of a test holder.
FIGS. 3A to 3D illustrate torsional registration fixtures that may be used to position the test device in an optical axis of a laser refractive system.
FIG. 4 illustrates an image of a test device taken by a camera of a wavefront sensor system.
FIGS. 5 and 6 illustrate images of the same test device taken by a camera of a laser refractive system.
FIG. 7 is a graph showing the torsional offset between the images of FIGS. 4 and 6.
FIG. 8 illustrates a kit.
FIG. 9 schematically illustrates a simplified method.

### DETAILED DESCRIPTION OF THE INVENTION

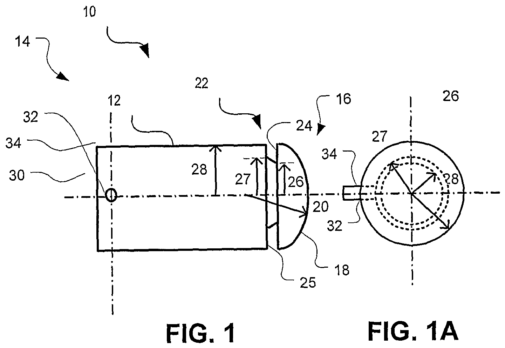

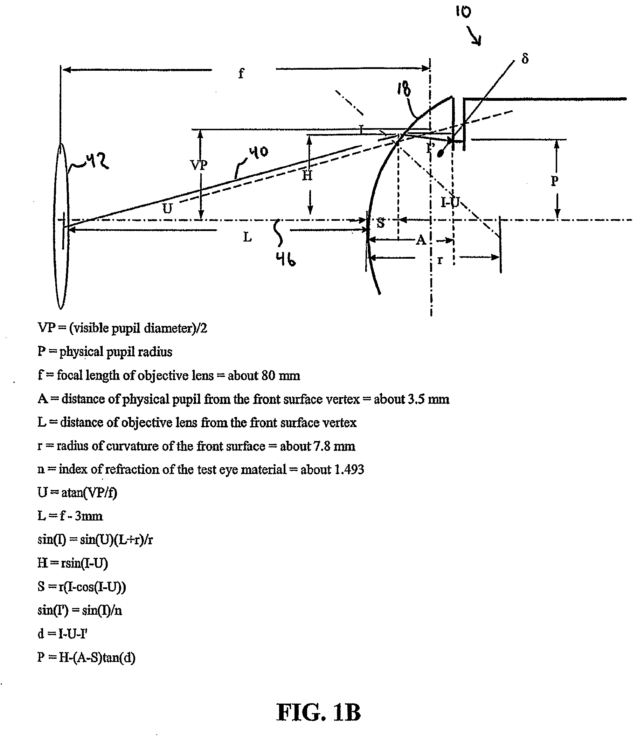

FIGS. 1 and 1A illustrate one embodiment of a test eye or test device 10. Test device 10 comprises a body 12 that has a distal end 14 and a proximal end 16. The illustrated embodiment of test device is substantially cylindrical, but it should be appreciated that the test device may also be such as substantially spherical, or the like.

Test device 10 may be comprised of a transparent optical material, such as cast polymethylmethacrylate (PMMA). Applicants have found that cast PMMA machines easily with a lathe and also provides a variation in its refractive index at different wavelengths (i.e., dispersion) that closely matches the index of refraction properties of the optical tissues of the human eye. While PMMA is one preferred material, other clear optical materials that can be ablated with a UV laser may be used. For example, glass may be used if spherical aberrations are desired to be incorporated into the test fixture.

Test device 10 may be composed of a single piece of material or may be composed of multiple pieces of machined pieces. A single piece test device 10 is preferred so that the test device 10 will be free of joints, thus ensuring a clear optical path.

Proximal end 16 of body 12 may include a radiused surface 18 that defines a proximal radius 20. In one embodiment, radiused surface has a radius of about 7.8 mm. Radiused surface 18 may have a curvature that coincides with a shape of a cornea of a human eye. Radiused surface 18 may have a smooth surface. If the surface is too rough, the diagnostic device may not be able to measure the surfaces of the test device.

Test fixture 10 may be manufactured to have a gap 22 in the proximal end 16 of body 12 adjacent the radiused surface 18. Gap 22 defines a front, annular shaped surface 24 and a back, annular shaped surface 25. The front, annular shaped surface 24 is substantially opposed to the radiused surface 18 on the proximal end 16 of body 12 and may be used to define an iris surface.

Test device 10 comprises a beveled surface within gap 22 in which a radius 27 measured from a longitudinal axis of test device, of beveled surface adjacent back, annular shaped surface 27 is larger than a radius 26 of the beveled surface adjacent the front, annular shaped surface 24. An outer radius 18 of body 12 will define an outermost radius of test device 10 and may be used to define an outer radius of the iris. The inner radius 26 of annular surface 24 may be used to define an inner surface of the iris and a pupil diameter.

The iris surface 24 may be manufactured so that it is rough and contains irregular marks/patches so as to provide a unique surface when imaged. Such irregular markings may be used by an alignment algorithm as "markers" so as to assist in finding a match for an eye and determining a torsional misalignment of the images of the test fixture taken by the different imaging devices (e.g., diagnostic device and laser refractive system). In some embodiments, iris surface 24 may be enhanced by scraping or otherwise treating iris surface 24 to create a realistic appearance of the iris in near infrared light. For example, a curved needle, carbon paper, carbon-based ink, such as a Sharpie pen or other comparable substances that are visible in infrared light, or the like, may be used to create texture patches, radial striations or other irregular types of imperfections on iris surface 24, so as to provide a surface that visually and optically reflects an iris of a human eye.

A distal surface 30 of body 12 maybe configured to provide a surface that acts as a retinal surface 30. Retinal surface 30 may be finished in a variety of ways to provide the retinal surface. In one embodiment, retinal surface 30 is treated to create a diffuse surface that absorbs light. For example, the retinal surface 30 may be treated with a single point diamond lathe finish to provide an optically flat surface. The retinal surface may be polished with a 0.3 micron Al₂O₃ grit to produce a diffuse back scattering surface, and thereafter painted a flat dark gray so as to absorb light that is directed through body 12. One paint that may be used to produce the flat dark gray is a "weather black" Floquil model railroad paint. As can be appreciated however, the present invention is not limited to this one embodiment and other types of treatments, polish, and paints may be used.

It should be appreciated that while the distal surface 30 is illustrated as a distal most surface, other surfaces within body 12 maybe treated to create a retinal surface, and the present invention is not limited to the retinal surface 30 being positioned on the distal most surface.

In some embodiments, a drilled hole 32 may be formed in the test fixture 10 at or near the distal end 14. Drilled hole 32 may have any diameter, but one preferred embodiment has a diameter of about 0.159 mm. In the illustrated embodiment, drilled hole 32 is about 4 mm away from the distal end of body 14. As can be appreciated, the distance the drilled hole 32 from the distal end may vary. An alignment pin 34 may be positioned within drilled hole 32 so as to provide a rotational alignment feature that is independent of the iris markings. The alignment pin 34 will have a diameter that substantially matches the diameter of the drilled hole 32 and may be press fit into drilled hole 32. In the illustrated embodiment, alignment pin comprises a stainless steel rod that is about 7 mm in length and extends radially from a longitudinal axis of body 12.

As may be appreciated, in other embodiments other conventional alignment references known in the art may be used to determine the torsional orientation of test fixture 10, such as a flattened surface, printed image, pins, cutoffs, flats, or the like. Furthermore, other embodiments of test fixture 10 may not need alignment mechanisms, since the cyclotorsional algorithms may use the iris markings to compare the torsional differences between images of the test fixture 10.

Referring now to FIG. 1B, embodiments of test device 10 typically have a geometry that provides a visible pupil size 2VP and a physical pupil size diameter 2P. The visible pupil size differs from the physical pupil size due to the magnification caused by the PMMA material. The following chart provides some examples of suitable diameters for a pupil of the test device 10:

| Visible pupil diameter (mm) | Physical pupil diameter (mm) |
|---|---|
| 3.000 | 2.559 |
| 4.000 | 3.424 |
| 5.000 | 4.301 |
| 6.000 | 5.194 |
| 7.000 | 6.109 |
| 8.000 | 7.054 |

As shown in FIG. 1B, due to the refractive properties and shape of the test device 10, the visible pupil diameter 2VP (i.e., the pupil size that the diagnostic device will image) differs from the physical pupil 2P diameter (i.e., the true, non-refractive pupil size). In particular, a principal ray 40 is shown passing from a center of the objective lens 42 of an imaging system to a radiused surface 18 of the test device 10 where it is refracted and passed into the test eye. Principal ray 40 strikes an edge of the physical pupil radius P at a distance P from the optical axis 46. The principal ray 40 is directed through test device 10 and makes an angle U with the optical axis 46 so that at one focal length f of the objective lens 42, its distance from the optical axis 46 is the visible pupil radius VP. The imaging system is assumed to be positioned so that the focal plane is 0.5 mm anterior to the plane of the physical pupil P. Distance P (e.g., physical pupil radius) is found by tracing this chosen ray through the optical system and finding where it strikes a plane at a distance A from the front surface vertex.

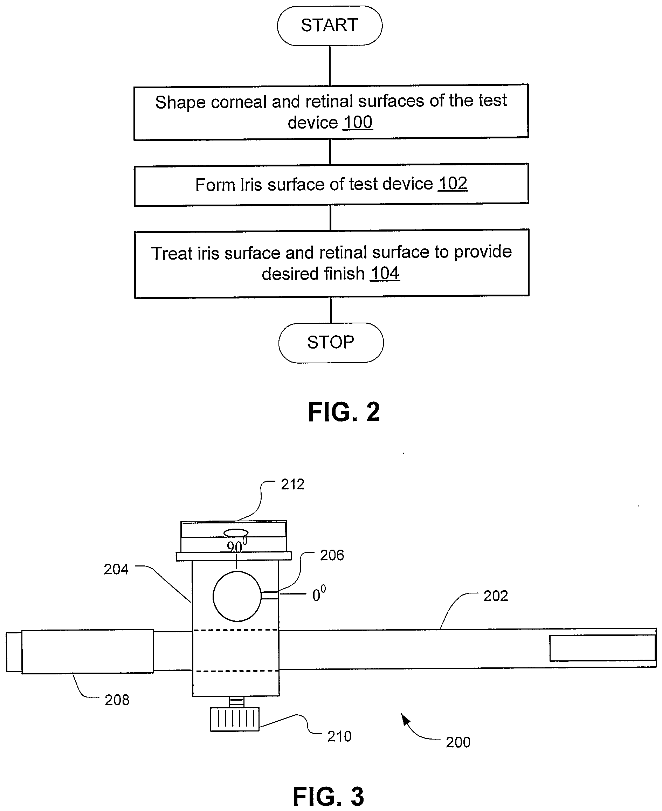

FIG. 2 schematically illustrates the method of manufacturing the test device 10. At 100, the method comprises shaping the body of the test device. The material may be shaped by creating the radiused front surface and a substantially flat back surface. The front surface may be shaped by a contact lens lathe (or other similar shaping devices) to create a smooth spherical surface that corresponds to the cornea of the human eye. Similarly, the back surface may be shaped to be optically flat. One exemplary contact lens lathe that may be used to create the front surface is ALM-OTT lathe system by DAC International of Capenteria, California.

At 102, the iris surface may be formed. The iris surface may be formed by creating an annular space in a proximal portion of the body 12 so as to create a front and back annular surface (FIG. 1).

At 104, the surfaces of the test device may be processed to create the desired characteristics. For example, the retinal surface may be polished to create an optically diffuse surface. The optically diffuse surface may thereafter be painted to create a surface that absorbs light. The corneal surface may be left untreated if it was originally created with a contact lens lathe. If however, the corneal surface was created with a conventional lathe, the corneal surface may be smoothed with a contact lens lathe to create an optically smooth spherical surface. To create the unique iris patches on the front annular surface 24 (FIG. 1), the iris surface may be treated with a conventional lathe. Additionally or alternatively, the iris surface may be enhanced with a material, such as a carbon paper, to scrape or scratch the iris surface to create radial striations, imperfections or other markings.

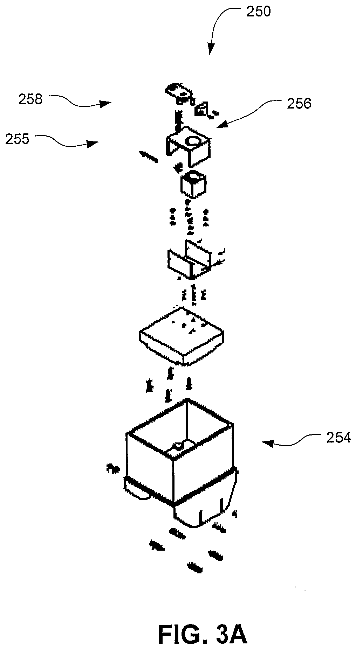

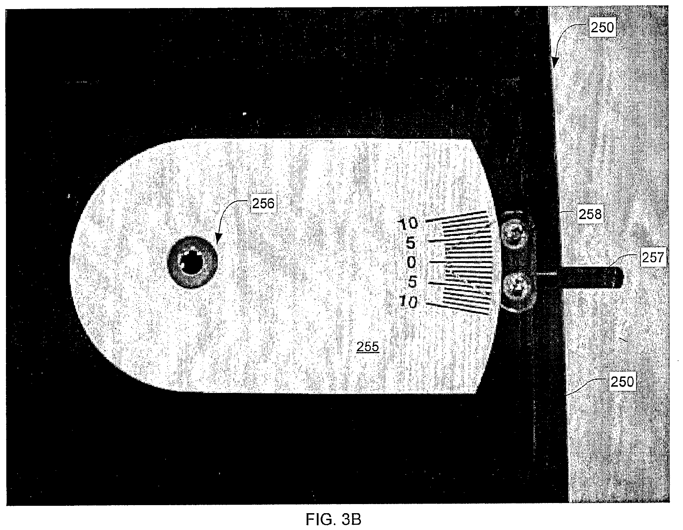

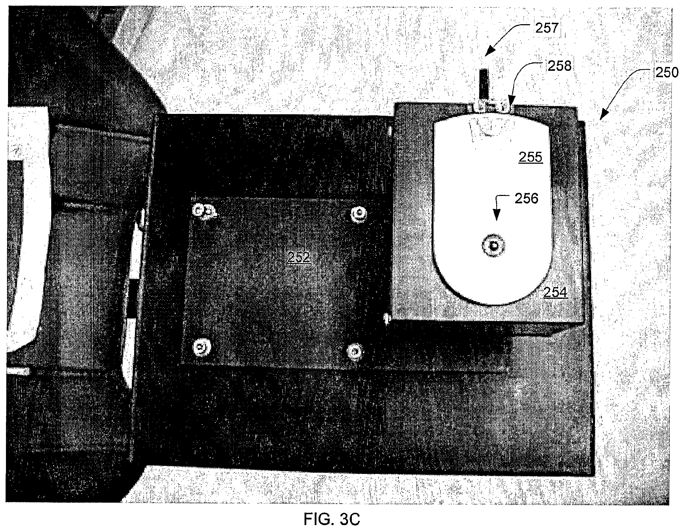

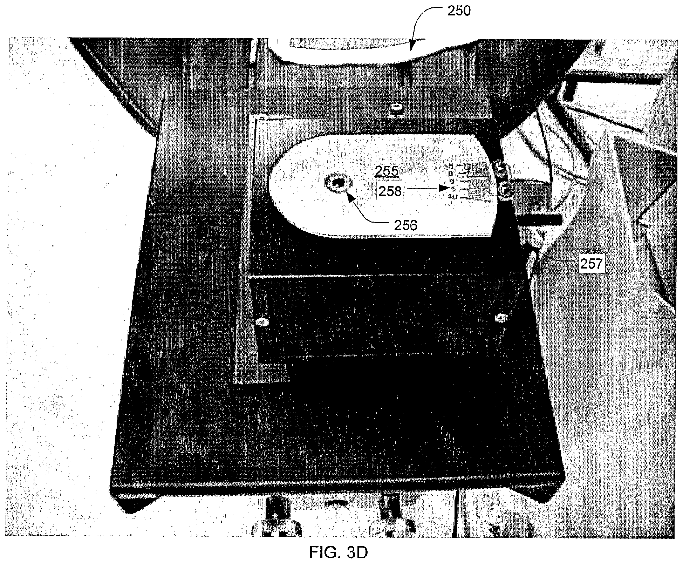

FIG. 3 illustrates a test device holder 200 that may be used to position the test device 10 in an optical path of an diagnostic device. In use, the user may examine the head rest support columns of the instrument (e.g., diagnostic device and/or laser refractive system) and determine how far from the support columns (in a horizontal direction), the normal eye location would be if a person's forehead was against the head rest and was looking into optical axis of the instrument. Some head-rest designs place the eye farther from the head-rest supports than others. If the proper eye position is close to the supports, it is best to place a round horizontal rod 202 of the test eye holder 200 on the side of the head-rest supports opposite from the instrument. If the eye position is much closer to the instrument than the head-rest supports, then it is best to place the horizontal rod 202 on the side of the supports closest to the instrument. height that vertically positions the test device 10 to a position where a patient's eye would typically be positioned. A rotatable platform 255 may be rotatably coupled to the base so as to allow the test device 10 to be rotated about an axis that coincides with the optical axis of the laser refractive system. Platform 255 may rotate the test device over any desired angles so as to impart a torsional misalignment. In the illustrated embodiment, the test device is allowed to be torsionally rotated ± 10 degrees. Platform 255 typically includes an opening 256 sized and shaped to receive the test device 10. Opening 256 may include an alignment slot (not shown) that receives the alignment pin 34 of the test device so as to position the test device 10 in a known orientation within registration fixture 250. Torsional registration fixture 250 may include a handle 257 and a torsional scale 258, such as a protractor, on its top surface that allows the user to torsionally position the test device 10 to a known torsional angle from its angle under the diagnostic device (i.e., 0 degrees).

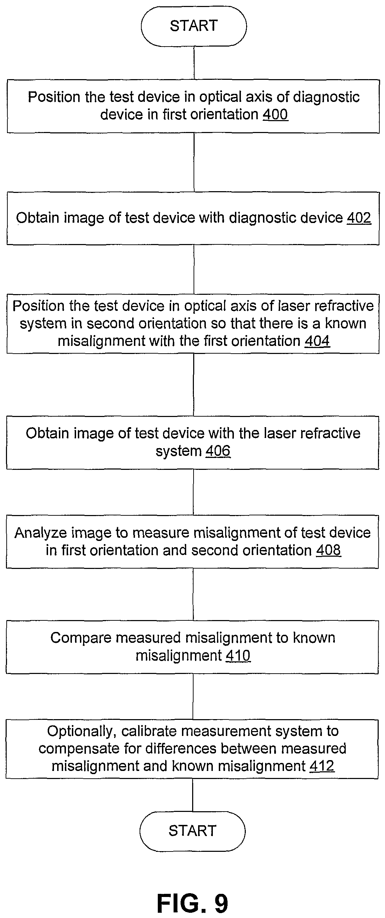

FIG. 9 illustrates schematically illustrates one embodiment of a method. At 400, the test device 10 is positioned in an optical axis of a diagnostic device in a first orientation. For the embodiments illustrated in FIG. 3, the alignment pin 34 of the test device is positioned in the 0 degree position. The 0 degree position in the test holder 200 may correspond to the 0 degree orientation in the registration fixture 250. At 402, the test device 10 is imaged by the diagnostic device. At 404, the test device is positioned in an optical axis of a laser refractive system. The test device 10 may be placed in a second orientation that is torsionally misaligned from the first orientation. For the embodiments illustrated in FIGS. 3A to 3D, the user may rotate platform 255 so that the test device is at a known torsional misalignment from the first orientation (i.e., any angle except the 0 degree angle, as shown by the readout on scale 258). Once the test device 10 is positioned at the desired torsional misalignment, an image of the misaligned test device may be obtained by the laser refractive system 406. At 408, the cyclotorsional algorithm may be used to measure the torsional misalignment between the test device in the first image and the test device in the second image. At 410, the measured torsional misalignment and the actual misalignment (as shown by scale 258) are compared to determine the accuracy of the cyclotorsional algorithm. The tests maybe repeated a plurality of times at a variety of different torsional misalignment angles to determine the accuracy of the cyclotorsional algorithms at different angles.

Thereafter, the cyclotorsional algorithm of the laser refractive system may be modified to compensate for the differences between the measured misalignment and the known misalignment, if desired.

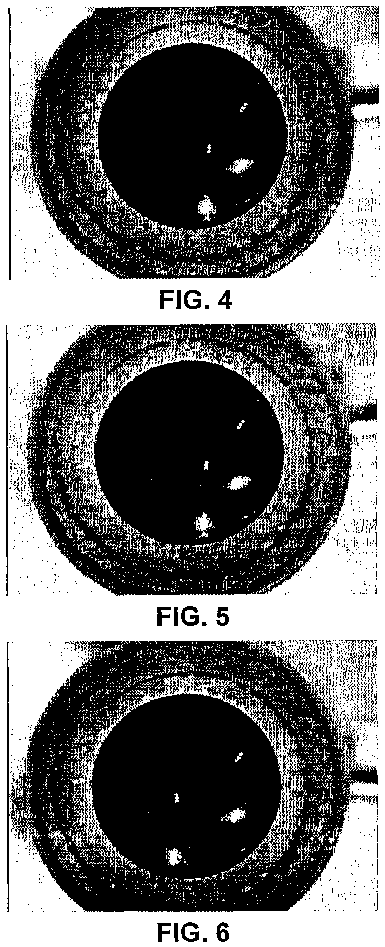

Appearance of the test device in an image of the diagnostic device and laser refractive system is shown in FIGS. 4 to 6. In particular, FIG. 4 illustrates test device 10 in an image taken by an eye camera of a wavefront instrument, such as the VISX WaveScan® System. FIGS. 5 and 6 are images of test device 10 that are obtained by a camera of a laser refractive system, such as the VISX STAR S4™ laser system. As shown in all three images, alignment pin 34 is visible in the images and acts as a fixed physical alignment reference that illustrates the difference in orientation of the test device in the different images of the two devices. Using the cyclotorsional algorithm,
the two images of the test device may be analyzed to determine the amount of torsional misalignment between the test device under the diagnostic device and the laser refractive system. A comparison of the actual and measured amount of torsional misalignment between the two images will tell the user how accurate the cyclotorsional algorithm is and thereafter allow the laser refractive system to be correctly calibrated for use in subsequent laser treatments.

A full description of one cyclotorsional algorithm is more fully described in co-pending and commonly owned U.S. Patent Application No. 10/300,714 filed November 19, 2002 (Publication No. US2003-0223037).
Because the test device has similar optical characteristics of a human eye and because the position of the test device 10 is placed in a position that the human eye would be in, the diagnostic device and laser refractive system do not have to be altered to determine the misalignment between the two instruments.

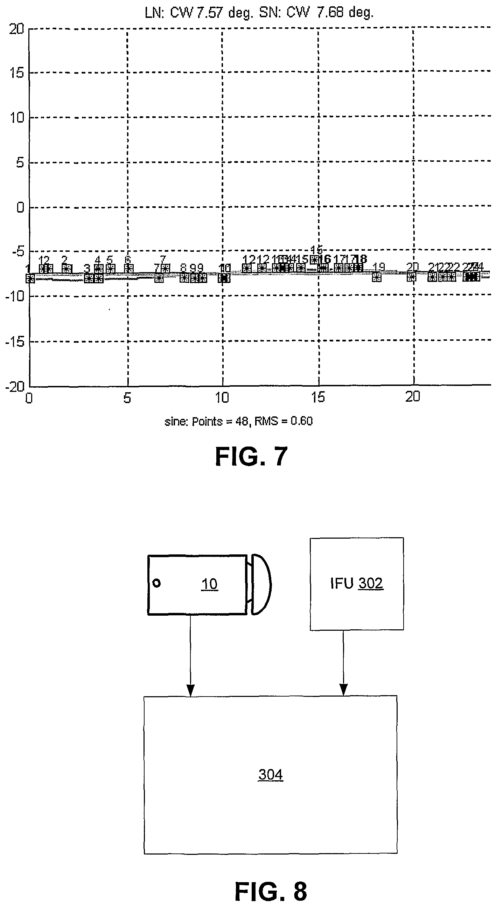

FIG. 7 shows an output of the cyclotorsional algorithm of an analysis of the images of FIGS. 4 and 6. The calculated amount of cyclotorsional rotation was computed to be about 7.68 degrees, which matched the actual cyclotorsional rotation to a precision of better than 1/4 degree. The cyclotorisional algorithm was based on matching patches of prominent iris texture between the two images from the diagnostic device and the laser refractive system. Because the manufacturing process of the test device 10 produces unique patterns of striations and imperfections on the iris, the algorithm is able to analyze test device 10 as it would a real eye.

FIG. 8 illustrates a kit. The kit 300 includes a test device 10 and instructions for use 302. The test device 10 and IFU 302 may be disposed within a packaging 304. Test device 10 will generally be as described above, and the instruction for use (IFU) 302 will set forth any of the methods described above. Package 304 may be any conventional medical device packaging, including pouches, trays, boxes, tubes, or the like. The instructions for use 302 will usually be printed on a separate piece of paper, but may also be printed in whole or in part on a portion of the package 304.

The kits may also include a torsional registration fixture 250 and a test eye holder 200 that positions the test device 10 in a path of at least one of the diagnostic device and the laser refractive system.

A variety of modifications are possible within the scope of the present invention. For example, the test device of the present invention may be ablated by the laser refractive system to create a known aberration into the surface of the test device. Thereafter, the test device may be imaged by a diagnostic device to test the diagnostic devices ability to measure the known aberrations. If the measurement from the diagnostic device matches the known aberration, the diagnostic device will not have to be calibrated. If however, the measurement from the diagnostic device does not match the known aberration, then the diagnostic device maybe calibrated. Moreover, once the aberration has been measured, the test device may be positioned in the optical axis of the laser refractive system so as to deliver a corrective ablation pattern to the test device. Advantageously, the ability of the cyclotorsional algorithm of the present invention to track the torsional alignment of the "eye" may be tested by positioning the test device in a torsionally misaligned orientation. After the corrective ablation pattern has been delivered to the test device, the test device may be placed in the optical axis of the diagnostic device to determine how well the ablation pattern was delivered.

Hence, the scope of the present application is not limited to the specifics of the embodiments described herein, but is instead limited solely by the appended claims.

## Claims

1. A test device for testing an alignment measurement between a diagnostic device and a laser refractive system, the device comprising:
a body (12) having visual and optical characteristics similar to that of a human eye, the body (12) comprising a proximal portion (16) and a distal portion (14);
wherein the proximal portion (16) defines a corneal surface (25) and a marked iris surface (24), and the distal portion (14) defines a retinal surface (30);
wherein the proximal position (16) of body (12) includes a radiused surface (18) that defines a proximal radius (20) and has a curvature that coincides with a shape of the corneal surface of a human eye; and
wherein the test device has a gap (22) in the proximal end (16) of the body (12) adjacent the radiused surface (18), the gap (22) defining a front, annular shaped surface (24) defining the marked iris surface (24) opposed to the radiused surface (18) and a back, annular shaped surface (25), defining the corneal surface, the test device (10) comprising a beveled surface within the gap (22) in which the beveled surface is of larger radius adjacent the back, annular shaped surface (27) than adjacent the front, annular shaped surface (24), wherein an outer radius (18) of the body (12) is arranged to define an outer radius of the iris and an inner radius (26) of annular surface (24) is arranged to define an inner surface of the iris and a pupil diameter.

2. The device of claim 1 wherein the corneal surface (25) is radiused.

3. The device of claim 1 wherein the iris surface (24) comprises one or more texture patches.

4. The device of claim 1 wherein the retinal surface (30) defines a diffuse surface.

5. The device of claim 4 wherein the diffuse surface absorbs light.

6. The device of claim 1 wherein the test device (10) defines a visible pupil diameter between about 3 mm and about 8 mm.

7. The device of claim 1 wherein the body (12) comprises a material that disperses different wavelengths of light at a substantially same rate as a human eye.

8. The device of claim 7 wherein the material comprises polymethylmethacrylate, PMMA, or glass.

9. The device of claim 1 wherein the body (12) further comprises an alignment reference (34).

10. The device of claim 9 wherein the alignment reference comprises an alignment pin (34) that extends radially from a longitudinal axis of the body (12).

11. The device of claim 7 wherein the alignment reference comprises a flattened surface along at least one of the proximal portion (16) and distal portion (14) of the body (12).

12. A method of manufacturing a test device (10), the method comprising:
providing a body (12) according to any of claim s 1 to 11;
treating the body (12) to form a textured annular iris surface (24), said surface comprising a pattern of striations and imperfections;
treating the first optical surface (30) to create a pupil surface.

13. The method of claim 12 wherein treating the first optical surface (30) comprises:
polishing the first optical surface (30) to produce a diffuse back scattering surface; and
applying a material that absorbs light to the polished surface.

14. The method of claim 13 wherein polishing is carried out with 0.3 micron Al₂O₃ grit.

15. The method of claim 13 wherein the material comprises a flat dark gray paint.

16. A method comprising:
positioning a test device (10) according to any of claims 1 to 11 in a first orientation;
obtaining a first image of the test device (10) with the diagnostic device;
positioning the test device (10) in an optical axis of a laser refractive system in a second orientation that is torsionally offset from the first orientation so as to provide a known torsional misalignment;
obtaining a second image of the test device (10) with the laser refractive system;
measuring a torsional misalignment of the test device (10) in the first image and the test device in the second image; and
comparing the measured misalignment with the known misalignment to determine the accuracy of the measured misalignment.

17. The method of claim 16 wherein the diagnostic device comprises an aberrometer.

18. The method of claim 17 wherein the aberrometer comprises a Hartmann-Shack device, Tscherning device, or a ray tracing device.

19. The method of claim 17 wherein the aberrometer comprises a wavefront measurement sensor.

20. The method of claim 16 wherein positioning the test device (10) within the optical axis comprises coupling the test device to a head rest (252).

21. The method of claim 16 wherein comparing the first image of the test device (10) to the second image of the test device (10) comprises determining a cyclotorsional rotation between the first image and the second image.

22. The method of claim 16 wherein the test device (10) is positioned within the optical axes of at least one of the diagnostic device and the laser refractive system with a holder.

23. The method of claim 16 wherein the measured misalignment is carried out with a cyclotorsional measurement algorithm.

24. The method of claim 22 further comprising calibrating the cyclotorsional measurement algorithm based on a difference between the measured misalignment and the known misalignment.

25. A kit for testing an alignment between a diagnostic device and a laser refractive system, the kit comprising:
a test device (10) according to any of claims 1 to 11;
instructions (302) for use comprising placing the test device (10) in a first orientation and obtaining a first image of the test device (10) with the diagnostic device, placing the test device (10) in a second orientation that is torsional offset from the first orientation, obtaining a second image of the test device (10) with the laser refractive system, and comparing a known torsional misalignment between the test device with a measured torsional misalignment that is calculated by comparing the first image of the test device to the second image of the test device; and
a package (304) to hold the test device and instructions for use.

## Patentansprüche

1. Prüfvorrichtung zum Prüfen einer Ausrichtungsmessung zwischen einer Diagnosevorrichtung und einem Laserbrechungssystem, die Vorrichtung umfassend:
einen Körper (12) mit visuellen und optischen Charakteristika ähnlich denen eines menschlichen Auges, der Körper (12) umfassend einen proximalen Abschnitt (16) und einen distalen Abschnitt (14);
wobei der proximale Abschnitt (16) eine Hornhautoberfläche (25) und eine markierte Irisoberfläche (24) definiert und der distale Abschnitt (14) eine Netzhautoberfläche (30) definiert;
wobei die proximale Position (16) des Körpers (12) eine gerundete Oberfläche (18) enthält, die einen proximalen Radius (20) definiert und eine Krümmung aufweist, die mit einer Form der Hornhautoberfläche eines menschlichen Auges übereinstimmt; und
wobei die Prüfvorrichtung einen Freiraum (22) in dem proximalen Ende (16) des Körpers (12) angrenzend an der gerundeten Oberfläche (18) aufweist, der Freiraum (22) eine die markierte Irisoberfläche (24) definierende vordere, ringförmig geformte Oberfläche (24) gegenüber der gerundeten Oberfläche (18) und eine die Hornhautoberfläche definierende hintere, ringförmig geformte Oberfläche (25) definiert,
die Prüfvorrichtung (10) umfassend eine abgeschrägte Oberfläche innerhalb des Freiraums (22), in dem die abgeschrägte Oberfläche einen größeren Radius aufweist, angrenzend an der hinteren, ringförmig geformten Oberfläche (27) anstatt angrenzend an der vorderen, ringförmig geformten Oberfläche (24), wobei ein äußerer Radius (18) des Körpers (12) angeordnet ist, einen äußeren Radius der Iris zu definieren, und ein innerer Radius (26) der ringförmigen Oberfläche (24) angeordnet ist, eine innere Oberfläche der Iris und einen Pupillendurchmesser zu definieren.

2. Vorrichtung nach Anspruch 1, wobei die Hornhautoberfläche (25) gerundet ist.

3. Vorrichtung nach Anspruch 1, wobei die Irisoberfläche (24) eine oder mehrere Texturstellen umfasst.

4. Vorrichtung nach Anspruch 1, wobei die Netzhautoberfläche (30) eine diffuse Oberfläche definiert.

5. Vorrichtung nach Anspruch 4, wobei die diffuse Oberfläche Licht absorbiert.

6. Vorrichtung nach Anspruch 1, wobei die Prüfvorrichtung (10) einen sichtbaren Pupillendurchmesser zwischen etwa 3 mm und etwa 8 mm definiert.

7. Vorrichtung nach Anspruch 1, wobei der Körper (12) ein Material umfasst, das verschiedene Wellenlängen von Licht bei einer im Wesentlichen gleichen Rate wie ein menschliches Auge zerstreut.

8. Vorrichtung nach Anspruch 7, wobei das Material Polymethylmethacrylat, PMMA, oder Glas umfasst.

9. Vorrichtung nach Anspruch 1, wobei der Körper (12) ferner eine Ausrichtungsreferenz (34) umfasst.

10. Vorrichtung nach Anspruch 9, wobei die Ausrichtungsreferenz einen Ausrichtungsstift (34) umfasst, der sich von einer Längsachse des Körpers (12) radial erstreckt.

11. Vorrichtung nach Anspruch 7, wobei die Ausrichtungsreferenz eine abgeflachte Oberfläche entlang mindestens einem des proximalen Abschnitts (16) und des distalen Abschnitts (14) des Körpers (12) umfasst.

12. Verfahren zum Herstellen einer Prüfvorrichtung (10), das Verfahren umfassend:
Bereitstellen eines Körpers (12) nach einem der Ansprüche 1 bis 11;
Behandeln des Körpers (12), um eine texturierte ringförmige Irisoberfläche (24) zu bilden, wobei die Oberfläche ein Muster von Streifungen und Unregelmäßigkeiten umfasst;
Behandeln der ersten optischen Oberfläche (30), um eine Pupillenoberfläche zu erzeugen.

13. Verfahren nach Anspruch 12, wobei Behandeln der ersten optischen Oberfläche (30) umfasst:
Polieren der ersten optischen Oberfläche (30), um eine diffuse hintere streuende Oberfläche zu produzieren; und
Auftragen eines Materials, das Licht absorbiert, auf die polierte Oberfläche.

14. Verfahren nach Anspruch 13, wobei Polieren mit einem Al₂O₃-Korn von 0,3 Mikrometer ausgeführt wird.

15. Verfahren nach Anspruch 13, wobei das Material eine matte dunkelgraue Farbe umfasst.

16. Verfahren, umfassend:
Positionieren einer Prüfvorrichtung (10) nach einem der Ansprüche 1 bis 11 in einer ersten Ausrichtung;
Erlangen eines ersten Bilds der Prüfvorrichtung (10) mit der Diagnosevorrichtung;
Positionieren der Prüfvorrichtung (10) in einer optischen Achse eines Laserbrechungssystems in einer zweiten Ausrichtung, die von der ersten Ausrichtung torsionsversetzt ist, um eine bekannte Torsionsfehlausrichtung bereitzustellen;
Erlangen eines zweiten Bilds der Prüfvorrichtung (10) mit dem Laserbrechungssystem;
Messen einer Torsionsfehlausrichtung der Prüfvorrichtung (10) in dem ersten Bild und der Prüfvorrichtung in dem zweiten Bild; und
Vergleichen der gemessenen Fehlausrichtung mit der bekannten Fehlausrichtung, um die Genauigkeit der gemessenen Fehlausrichtung zu bestimmen.

17. Verfahren nach Anspruch 16, wobei die Diagnosevorrichtung einen Abweichungsmesser umfasst.

18. Verfahren nach Anspruch 17, wobei der Abweichungsmesser eine Hartmann-Shack-Vorrichtung, eine Tscherning-Vorrichtung oder eine Strahlenverfolgungsvorrichtung umfasst.

19. Verfahren nach Anspruch 17, wobei der Abweichungsmesser einen Wellenfrontmessungssensor umfasst.

20. Verfahren nach Anspruch 16, wobei Positionieren der Prüfvorrichtung (10) in der optischen Achse umfasst, die Prüfvorrichtung an eine Kopfstütze (252) zu koppeln.

21. Verfahren nach Anspruch 16, wobei Vergleichen des ersten Bilds der Prüfvorrichtung (10) mit dem zweiten Bild der Prüfvorrichtung (10) umfasst, eine Cyclotorsionsrotation zwischen dem ersten Bild und dem zweiten Bild zu bestimmen.

22. Verfahren nach Anspruch 16, wobei die Prüfvorrichtung (10) in den optischen Achsen von mindestens einem der Diagnosevorrichtung und des Laserbrechungssystems mit einem Halter positioniert ist.

23. Verfahren nach Anspruch 16, wobei die gemessene Fehlausrichtung mit einem Cyclotorsionsmessalgorithmus ausgeführt wird.

24. Verfahren nach Anspruch 22, ferner umfassend, den Cyclotorsionsmessalgorithmus basierend auf einer Differenz zwischen der gemessenen Fehlausrichtung und der bekannten Fehlausrichtung zu kalibrieren.

25. Kit zum Prüfen einer Fehlausrichtung zwischen einer Diagnosevorrichtung und einen Laserbrechungssystem, der Kit umfassend:
eine Prüfvorrichtung (10) nach einem der Ansprüche 1 bis 11;
eine Gebrauchsanleitung (302), umfassend Platzieren der Prüfvorrichtung (10) in einer ersten Ausrichtung und Erlangen eines ersten Bilds der Prüfvorrichtung (10) mit der Diagnosevorrichtung, Platzieren der Prüfvorrichtung (10) in einer zweiten Ausrichtung, die von der ersten Ausrichtung torsionsversetzt ist, Erlangen eines zweiten Bilds der Prüfvorrichtung (10) mit dem Laserbrechungssystem und Vergleichen einer bekannten Torsionsfehlausrichtung zwischen der Prüfvorrichtung mit einer gemessenen Torsionsfehlausrichtung, die durch Vergleichen des ersten Bilds der Prüfvorrichtung mit dem zweiten Bild der Prüfvorrichtung berechnet wird; und
eine Packung (304) zum Halten der Prüfvorrichtung und der Gebrauchsanleitung.

## Revendications

1. Dispositif de test pour tester une mesure d'alignement entre un dispositif de diagnostic et un système réfractif laser, le dispositif comprenant :
un corps (12) ayant des caractéristiques visuelles et optiques similaires à celles d'un oeil humain, le corps (12) comprenant une partie proximale (16) et une partie distale (14) ;
où la partie proximale (16) définit une surface cornéenne (25) et une surface d'iris marquée (24), et la partie distale (14) définit une surface rétinienne (30) ;
où la position proximale (16) du corps (12) comprend une surface arrondie (18) qui définit un rayon proximal (20) et possède une courbure qui coïncide avec la forme de la surface cornéenne d'un oeil humain ; et
où le dispositif de test comporte un espace (22) dans l'extrémité proximale (16) du corps (12) adjacent à la surface arrondie (18), l'espace (22) définissant une surface frontale de forme annulaire (24) définissant à la surface d'iris marquée (24) opposée à la surface arrondie (18) et une surface arrière de forme annulaire (25), définissant la surface cornéenne, le dispositif de test (10) comprenant une surface biseautée dans l'espace (22) où la surface biseautée a un rayon adjacent à la surface arrière de forme annulaire (27) plus grand que le rayon adjacent à la surface frontale de forme annulaire (24), où un rayon extérieur (18) du corps (12) est arrangé pour définir un rayon extérieur de l'iris, et un rayon intérieur (26) d'une surface annulaire (24) est arrangé pour définir une surface intérieure de l'iris et d'un diamètre de pupille.

2. Dispositif selon la revendication 1, dans lequel la surface cornéenne (25) est arrondie.

3. Dispositif selon la revendication 1, dans lequel la surface d'iris (24) comprend une ou plusieurs pièces de texture.

4. Dispositif selon la revendication 1, dans lequel la surface rétinienne (30) définit une surface diffuse.

5. Dispositif selon la revendication 4, dans lequel la surface diffuse absorbe la lumière.

6. Dispositif selon la revendication 1, dans lequel le dispositif de test (10) définit un diamètre de pupille visible compris entre environ 3 mm et environ 8 mm.

7. Dispositif selon la revendication 1, dans lequel le corps (12) comprend un matériau qui disperse différentes longueurs d'onde de lumière à un taux sensiblement identique à un oeil humain.

8. Dispositif selon la revendication 7, dans lequel le matériau comprend du polyméthacrylate de méthyle, du PMMA, ou du verre.

9. Dispositif selon la revendication 1, dans lequel le corps (12) comprend en outre une référence d'alignement (34).

10. Dispositif selon la revendication 9, dans lequel la référence d'alignement comprend une broche d'alignement (34) qui s'étend radialement depuis un axe longitudinal du corps (12) .

11. Dispositif selon la revendication 7, dans lequel la référence d'alignement comprend une surface aplatie le long d'au moins une partie parmi la partie proximale (16) et la partie distale (14) du corps (12).

12. Procédé de fabrication d'un dispositif de test (10), le procédé comprenant les étapes suivantes :
fournir un corps (12) selon l'une quelconque des revendications 1 à 11 ;
traiter le corps (12) pour former une surface d'iris annulaire texturée (24), ladite surface comprenant un motif de stries et d'imperfections ;
traiter la première surface optique (30) pour créer une surface de pupille.

13. Procédé selon la revendication 12, dans lequel traiter la première surface optique (30) comprend les étapes suivantes :
polir la première surface optique (30) pour produire une surface de dispersion arrière diffuse ; et
appliquer un matériau qui absorbe la lumière à la surface polie.

14. Procédé selon la revendication 13, dans lequel le polissage est effectué avec un abrasif d'Al₂O₃ de 0,3 micron.

15. Procédé selon la revendication 13, dans lequel le matériau comprend une peinture mate grise foncée.

16. Procédé comprenant les étapes suivantes :
positionner un dispositif de test (10) selon l'une quelconque des revendications 1 à 11 suivant une première orientation ;
obtenir une première image du dispositif de test (10) avec le dispositif de diagnostic ;
positionner le dispositif de test (10) dans un axe optique d'un système réfractif laser dans une seconde orientation qui est décalée en torsion par rapport à la première orientation de manière à présenter un défaut d'alignement de torsion connu ;
obtenir une seconde image du dispositif de test (10) avec le système réfractif laser ;
mesurer un défaut d'alignement de torsion du dispositif de test (10) dans la première image et du dispositif de test dans la seconde image ; et
comparer le défaut d'alignement mesuré au défaut d'alignement connu pour déterminer la précision du défaut d'alignement mesuré.

17. Procédé selon la revendication 16, dans lequel le dispositif de diagnostic comprend un aberromètre.

18. Procédé selon la revendication 17, dans lequel l'aberromètre comprend un dispositif de Hartmann-Shack, un dispositif de Tscherning ou un dispositif de traçage de rayon.

19. Procédé selon la revendication 17, dans lequel l'aberromètre comprend un détecteur de mesure de front d'onde.

20. Procédé selon la revendication 16, dans lequel positionner le dispositif de test (10) sur l'axe optique comprend de coupler le dispositif de test à un appui-tête (252) .

21. Procédé selon la revendication 16, dans lequel comparer la première image du dispositif de test (10) à la seconde image du dispositif de test (10) comprend de déterminer une rotation de cyclotorsion entre la première image et la seconde image.

22. Procédé selon la revendication 16, dans lequel le dispositif de test (10) est positionné sur les axes optiques d'au moins l'un du dispositif de diagnostic et du système réfractif laser avec un support.

23. Procédé selon la revendication 16, dans lequel le défaut d'alignement mesuré est relevé avec un algorithme de mesure de cyclotorsion.

24. Procédé selon la revendication 22, comprenant en outre d'étalonner l'algorithme de mesure de cyclotorsion sur la base d'une différence entre le défaut d'alignement mesuré et le défaut d'alignement connu.

25. Kit pour tester un défaut d'alignement entre un dispositif de diagnostic et un système réfractif laser, le kit comprenant :
un dispositif de test (10) selon l'une quelconque des revendications 1 à 11 ;
des instructions (302) d'utilisation comprenant de placer le dispositif de test (10) suivant une première orientation et d'obtenir une première image du dispositif de test (10) avec le dispositif de diagnostic, de placer le dispositif de test (10) suivant une seconde orientation qui présente un décalage de torsion par rapport à la première orientation, d'obtenir une seconde image du dispositif de test (10) avec le système réfractif laser, et de comparer un défaut d'alignement de torsion connu entre le dispositif de test avec un défaut d'alignement mesuré qui est calculé en comparant la première image du dispositif de test à la seconde image du dispositif de test ; et
un boîtier (304) pour contenir le dispositif de test et des instructions d'utilisation.
